# EUROPEAN PATENT SPECIFICATION

(11) **EP 1 466 645 B1**
(45) Date of publication and mention of the grant of the patent: **22.08.2007**
(21) Application number: 04008646.4
(22) Date of filing: 08.04.2004
(51) Int. Cl.: A61M 25/06, A61M 25/00, A61M 39/10, A61M 39/14, A61M 25/02, A61M 5/32

(54) **Connector set for medical use and indwelling catheter set using such connector set**
Medizintechnischer Verbindersatz und Verweilkatheter
connecteur médical et cathéter à demeure

(30) Priority: 08.04.2003 JP 2003103827
(43) Date of publication of application: 13.10.2004
(73) Proprietor: NIPRO CORPORATION, Kita-ku, Osaka-shi, Osaka-fu, 531-8510 (JP)
(72) Inventor: Yashiro, Kenji c/o NIPRO MEDICAL INDUSTRIES LTD., Tatebayashi-shi Gunma-ken 374-8518 (JP); Higaki, Yoshio c/o NIPRO MEDICAL INDUSTRIES LTD., Tatebayashi-shi Gunma-ken 374-8518 (JP); Yano, Tsuyoshi c/o NIPRO MEDICAL INDUSTRIES LTD., Tatebayashi-shi Gunma-ken 374-8518 (JP)
(74) Representative: Banzer, Hans-Jörg

(56) References cited:
- WO-A-02/07804
- WO-A-90/01349
- US-A- 3 906 932
- US-A- 5 976 115
- US-A1- 2001 047 154

## Description

### Field of the Invention

The present invention relates to various connector sets for medical use provided, for example, on a solution infusion line, a blood transfusion line, a blood collection line, and an arterial and venous line for dialysis therapy, and to various indwelling catheter sets for performing solution infusion, blood transfusion, blood collection, and dialysis therapy, and so on (for example, a hypodermic injection set for a drug solution such as an insulin administration set).

### Description of the Related Art

There is an administration set for administering insulin discontinuously or continuously having a female luer taper, and a needle and a male luer taper to be detachably connected to the female luer taper respectively (for example, see USP NO.6056718).

In this set, the female luer taper includes:
(a) a catheter disposed in the axial direction and formed into a hollow body having openings at its distal and proximal ends;
(b) a catheter hub including an axially extending through bore having an opening at its distal end for fitting the catheter and an opening at its proximal end as a connecting port for feeding insulin; and
(c) a plug formed of rubber material to be fitted to the connecting port for sealing the connecting port.

The needle includes:
(a) an insertion needle disposed in the axial direction, the insertion needle being detachably inserted into the plug and the catheter from proximal ends thereof when connecting the needle and the female luer taper; and
(b) an insertion needle hub provided at a proximal end of the insertion needle, the insertion needle hub being detachably connected to the catheter hub from its proximal end when connecting the needle and the female luer taper.

The male luer taper includes:
(a) an infusion needle disposed in the axial direction and formed into a hollow body having openings at its distal and proximal end, the infusion needle being connected to the insulin administering line at its proximal end, the infusion needle being detachably inserted into the plug from its proximal end when connecting the male luer taper and the female luer taper, so as to communicate with the catheter; and
(b) an infusion tubing hub provided at the proximal portion of the infusion needle, the infusion tubing hub being detachably connected to the catheter hub from its proximal end when connecting the male luer taper and the female luer taper.

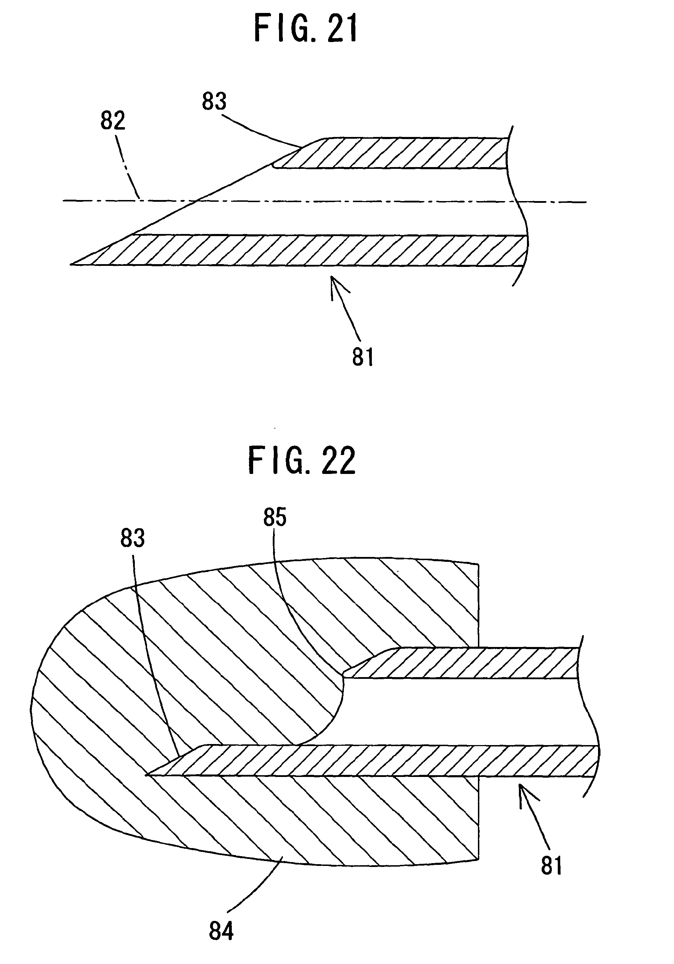

As shown in Fig. 21, a distal end of an infusion needle 81 is cut obliquely with respect to an axial center 82 thereof (bevel cut), and hence a distal opening edge 83 is beveled to provide a sharp cutting edge at its distal end, so that the infusion needle 81 itself has a puncturing capability with respect to the plug.

In the configuration described above, when indwelling the catheter under the skin of a patient, the needle is connected to the female luer taper, and the insertion needle of the needle and the catheter of the female luer taper are inserted under the skin of the patient and, subsequently, the insertion needle is pulled out from under the skin of the patient by detaching the needle from the female luer taper so that the catheter is left under the skin of the patient. In this case, since the connecting port of the female luer taper is sealed by the plug, there is no risk of leakage of blood from the connecting port.

Then, what has to be done when administering insulin discontinuously or continuously is to connect the male luer taper to the female luer taper, insert the infusion needle of the male luer taper into the plug of the female luer taper so as to communicate with the catheter, and administer insulin to the patient. When administration is completed, the male luer taper is disconnected from the female luer taper, and the infusion needle of the male luer taper is pulled out from the plug of the female luer taper. In this case as well, since the connecting port of the female luer taper is sealed by the plug, there is no risk of leakage of blood from the connecting port. Since administration of insulin is carried out as needed according to the conditions of the patient, the infusion needle of the male luer taper may be inserted into the plug of the female luer taper a plurality of times.

In USP No. 6056718, as described above (see Fig. 21), the distal end of the infusion needle 81 is cut obliquely with respect to the axial center 82 thereof to provide a sharp cutting edge, so that the infusion needle 81 itself has a puncturing capability with respect to the plug.

Therefore, when inserting the infusion needle to the plug, the possibility that the infusion needle can follow the trace (puncture) formed on the plug by the insertion needle when it is inserted therein previously is extremely low, and in many cases, the distal end formed into a sharp cutting edge punctures into the plug afresh and is advanced therein while tearing the plug, and then the proximal portion thereof is followed into the plug in sequence. Accordingly, as shown in Fig. 22, the portion of the plug 84 located on the distal side of the infusion needle 81 enters within the infusion needle 81, and is subjected to cutting operation by an inner peripheral edge 85 on the proximal side of the opening edge 83 at the distal end of the infusion needle 81.

Therefore, after repeated insertion of the infusion needle into the plug, the plug is ground off, and such a phenomenon that a bore (puncture) is formed in the plug (coring) occurs. Consequently, the performance of the plug to seal the connecting port is deteriorated and, in addition, "ground chips" of the plug may enter into the infusion needle or the catheter, and eventually into the patient's body.

In addition, since the distal end of the infusion needle is formed into a sharp cutting edge and hence itself has a puncturing capability with respect to the plug as described above, the infusion needle is inserted into the different points of the plug at every insertion into the plug, and hence tearing of the plug tends to take place. Therefore, when insertion of the infusion needle into the plug is repeated, the plug may lose its original strength or recovering property, and hence the performance of the plug to seal the connecting port may be deteriorated. Also, as described above, since the plug is repeatedly torn, fine chips of the plug tend to be generated, which may enter into the patient's body as in the above-described case.

Furthermore, when the distal end of the infusion needle is formed into a sharp cutting edge as described above, the user has a risk to prick his/her finger or hand accidentally by the infusion needle. In order to avoid such risk, it is necessary to provide a protective wall which surrounds the infusion needle as disclosed in USP NO. 6056718. However, in doing so, another problem that the male luer taper becomes bulky arises.

WO 02/07804 A, which serves as basis for the preamble of claim 1, discloses an infusion set comprising a cannula housing, a needle housing adapted for connection to the cannula housing, and a needle, the cannula housing having a bore in which a self-sealing elastomeric septum is disposed. In use, the needle which has a pointed end extends through a self-closing aperture in the septum. The needle delivers fluid from a fluid source to a cannula extending from and connected to the cannula housing.

US 2001/0047154 A1 relates to a pre-slit injection site and tapered cannula and discloses two-part coupling members for fluid transfer including a pre-slit septum and a blunt cannula. The blunt cannula may be forced through the septum in order to establish a fluid communication path. An exterior surface of a distal end region of the cannula may be tapered, the end of the distal end region including an externally radiused tip.

### Brief Summary of the Invention

As a result of earnest investigations made by the inventors to solve the problems associated with the conventional techniques, the present invention has been completed.

It is a first object of the present invention to provide a connector set for medical use, in which the possibility of deterioration of the performance of the plug to seal the connecting port is significantly low even when insertion of the infusion needle into the plug is repeated, and the possibility of entering of "ground chips" or fine strips of the plug into the patient's body is significantly low, and to provide an indwelling catheter set using such connector set.

It is a second object of the present invention to provide a connector set for medical use, in which the risk that the user pricks his/her finger or hand accidentally by the infusion needle is extremely low and hence it is not necessary to provide a protective wall surrounding the infusion needle so that the male luer taper may be downsized, and an indwelling catheter set using such connector set.

The above-mentioned objects and other objects of the present invention will be clarified further more in the following description, and these objects are attained by the present invention comprising the constitution mentioned below.

According to the present invention, a connector set for medical use as defined by independent claim 1 and an indwelling catheter set using such connector set as defined by claim 7 are provided. The dependent claims define preferred and advantageous embodiments of the invention.

The present invention relates to a connector set for medical use comprising:
(a) a cannula housing; and
(b) an infusion hub to be detachably connected to the cannula housing from its proximal end,
the cannula housing comprising:
(a) a catheter hub, a proximal portion of which is formed into a connecting port for feeding liquid; and
(b) a plug formed of resilient material for sealing the connecting port, the plug being inserted into the connecting port,
the infusion hub comprising:
(a) an infusion needle for feeding liquid disposed substantially in the axial direction and formed into a hollow body opened at its distal and proximal ends, the infusion needle being detachably inserted into the plug from its proximal end when connecting the infusion hub and the cannula housing; and
(b) an infusion tubing hub provided at a proximal portion of the infusion needle, the infusion tubing hub being detachably connected to the catheter hub from its proximal end when connecting the infusion hub and the cannula housing,
wherein an opening edge of a distal end of the infusion needle is orthogonal to an axial center of the infusion needle, an inner peripheral side and an outer peripheral side of the opening edge being respectively formed into an outwardly projecting curved surface.

The connector set for medical use may further comprise:
an insertion hub to be detachably connected to the cannula housing from its proximal end,
the insertion hub comprising:
   (a) an insertion needle disposed substantially in the axial direction, the insertion needle being detachably inserted into the plug from its proximal end when connecting the insertion hub and the cannula housing; and
   (b) an insertion needle hub provided at a proximal portion of the insertion needle, the insertion needle hub being detachably connected to the catheter hub from its proximal end when connecting the insertion hub and the cannula housing.

The connecter set for medical use may further comprise:
a guide formed into a hollow body opened at its distal and proximal ends for preventing dropping off of the plug from the connecting port,
the guide being inserted into a portion of the connecting port, which is disposed proximally of the plug,
the guide allowing detachable insertion of the infusion needle from its proximal end,
the guide being formed with a tapered bore having a distally tapered shape at least at a proximal portion in the interior of the guide,
an inner surface of the tapered bore being configured to guide the infusion needle toward a substantially axial center of the connecting port.

The guide may comprise in the interior:
(a) a straight bore forming a distal portion of the interior of the guide and having an inner diameter substantially constant in the axial direction; and
(b) the tapered bore forming the proximal portion of the interior of the guide.

The infusion needle and the infusion tubing hub may be integrally formed of plastic material,
the opening edge of the distal end of the infusion needle being formed to be orthogonal to the axial center of the infusion needle and the inner peripheral side and the outer peripheral side of the opening edge being respectively formed into the outwardly projecting curved surface at the time of molding.

The infusion needle may be coated with lubricant applied thereon.

The present invention also relates to an indwelling catheter set comprising the above-described connector set,
wherein a through bore is formed in the catheter hub of the cannula housing substantially in the axial direction,
a proximal portion of the through bore being formed into the connecting port,
a catheter being inserted into a distal portion of the through bore,
the catheter being disposed substantially in the axial direction, and being formed into a hollow body opened at its distal and proximal ends,
the catheter being communicated with the infusion needle when the infusion needle is inserted into the plug.

The indwelling catheter set may comprise:
(a) a cannula housing; and
(b) an insertion hub and an infusion hub to be detachably connected to the cannula housing from its proximal end respectively,
the cannula housing comprising:
(a) a catheter disposed substantially in the axial direction and formed into a hollow body opened at its distal and proximal ends;
(b) a catheter hub comprising a through bore formed substantially in the axial direction, the through bore comprising a distal portion for allowing insertion of the catheter and a proximal portion serving as a connecting port for feeding liquid; and
(c) a plug formed of resilient material for sealing the connecting port by being inserted into the connecting port,
the insertion hub comprising:
(a) an insertion needle disposed substantially in the axial direction, the insertion needle being detachably inserted into the plug and the catheter from proximal ends thereof when connecting the insertion hub and the cannula housing; and
(b) an insertion needle hub provided at a proximal portion of the insertion needle, the insertion needle hub being detachably connected to the catheter hub from its proximal end when connecting the insertion hub and the cannula housing,
the infusion hub comprising:
(a) an infusion needle for feeding liquid disposed substantially in the axial direction and formed into a hollow body opened at its distal and proximal ends, the infusion needle being detachably inserted into the plug from its proximal end, when connecting the infusion hub and the cannula housing, so as to communicate with the catheter; and
(b) an infusion tubing hub provided at a proximal portion of the infusion needle, the infusion tubing hub being detachably connected to the catheter hub from its proximal end when connecting the infusion hub and the cannula housing,
wherein an opening edge of a proximal end of the infusion needle is orthogonal to an axial center of the infusion needle,
an inner peripheral side and an outer peripheral side of the opening edge being respectively formed into an outwardly projecting curved surface.

### Brief Description of the Drawings

Fig. 1 to Fig. 16 show a first embodiment of the present invention, and Fig. 1 is a plan view.
Fig. 2 is a plan view, partly in cross section, of Fig. 1.
Fig. 3 is a vertical cross section of Fig. 2.
Fig. 4 is a partly enlarged view of Fig. 3.
Fig. 5 is an exploded view of Fig. 3.
Fig. 6 is a plan view, partly in cross section, of a cannula housing in Fig. 2.
Fig. 7 is a partly enlarged view of the cannula housing in Fig. 5.
Fig. 8A is a side view of a guide in Fig. 7, Fig. 8B is a vertical cross section of Fig 8A.
Fig. 9 is a plan view, partly in cross section, of an insertion hub in Fig. 2.
Fig. 10 is a plan view showing a state in which the insertion hub in Fig. 1 is replaced by an infusion hub.
Fig. 11 is a plan view, partly in cross section, of Fig. 10.
Fig. 12 is a vertical cross section of Fig. 11.
Fig. 13 is a plan view, partly in cross section, of a infusion hub in Fig. 11.
Fig. 14 is a vertical cross section of Fig. 13.
Fig. 15 is an enlarged view of a distal end of an infusion needle in Fig. 14.
Fig. 16 is a vertical cross section showing the operation of the infusion needle in Fig. 15.
Fig. 17 and Fig. 18 show a second embodiment of the present invention, and Fig. 17 is a vertical cross section.
Fig. 18A is a side view of a guide in Fig. 17, Fig. 18B is a vertical cross section of Fig 18A.
Fig. 19 and Fig. 20 show a third embodiment of the present invention, and Fig. 19 is a plan view, partly in cross section.
Fig. 20 is a vertical cross section of Fig. 19.
Fig. 21 and Fig. 22 show an example in the related art and Fig. 21 is a vertical cross section of the infusion needle.
Fig. 22 is a vertical cross section showing the operation of the infusion needle in Fig. 21.

### Detailed Description of the Invention

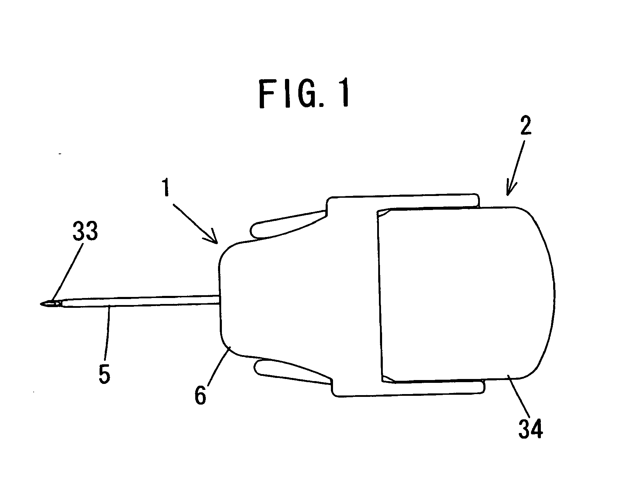

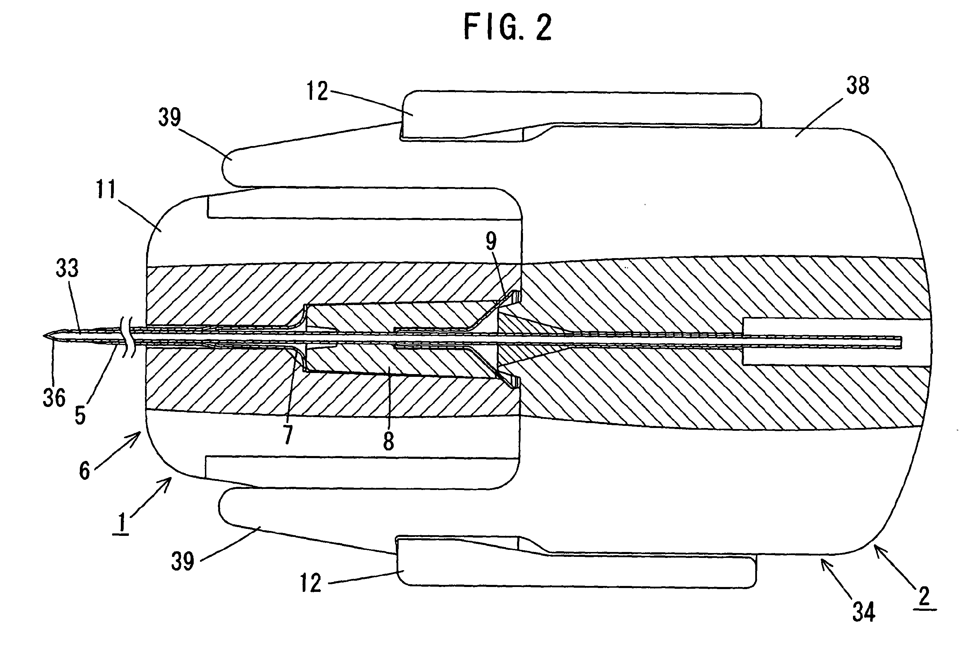

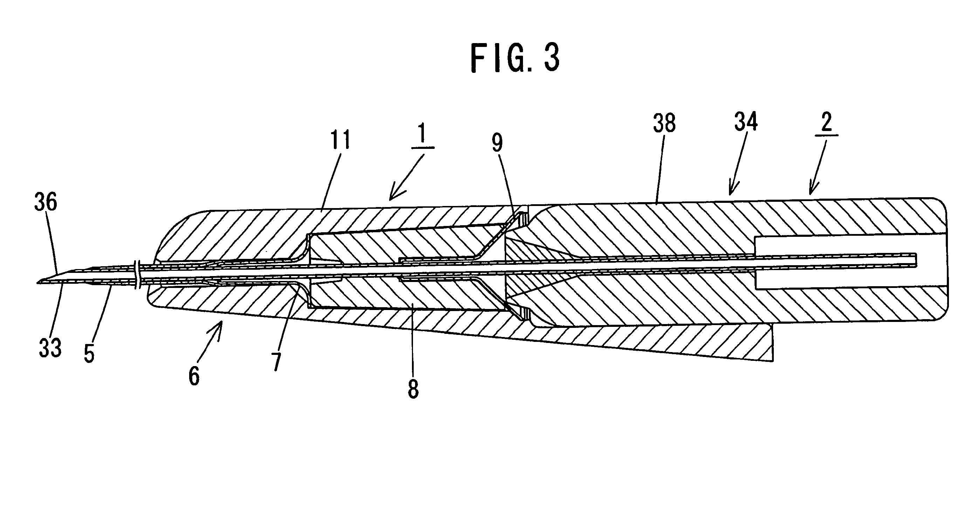

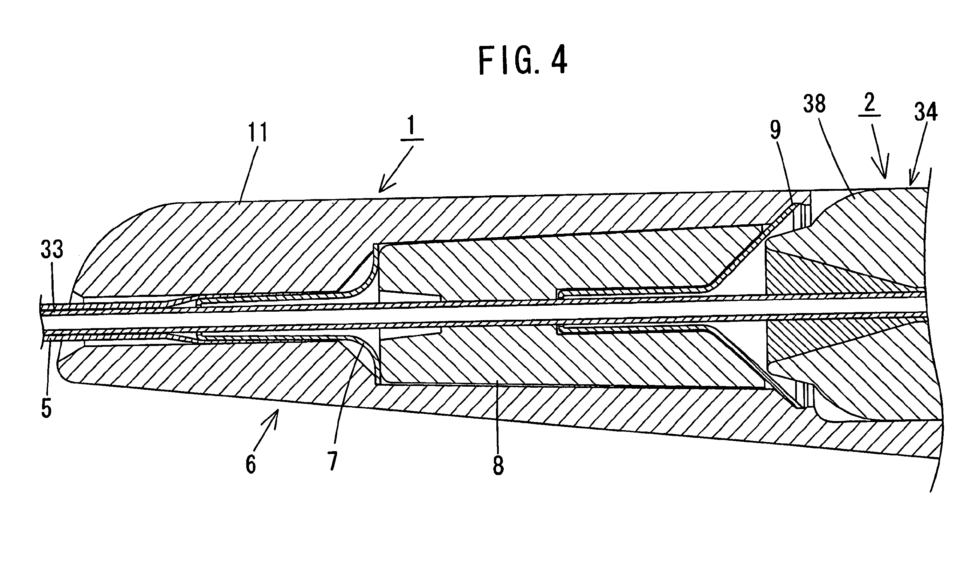

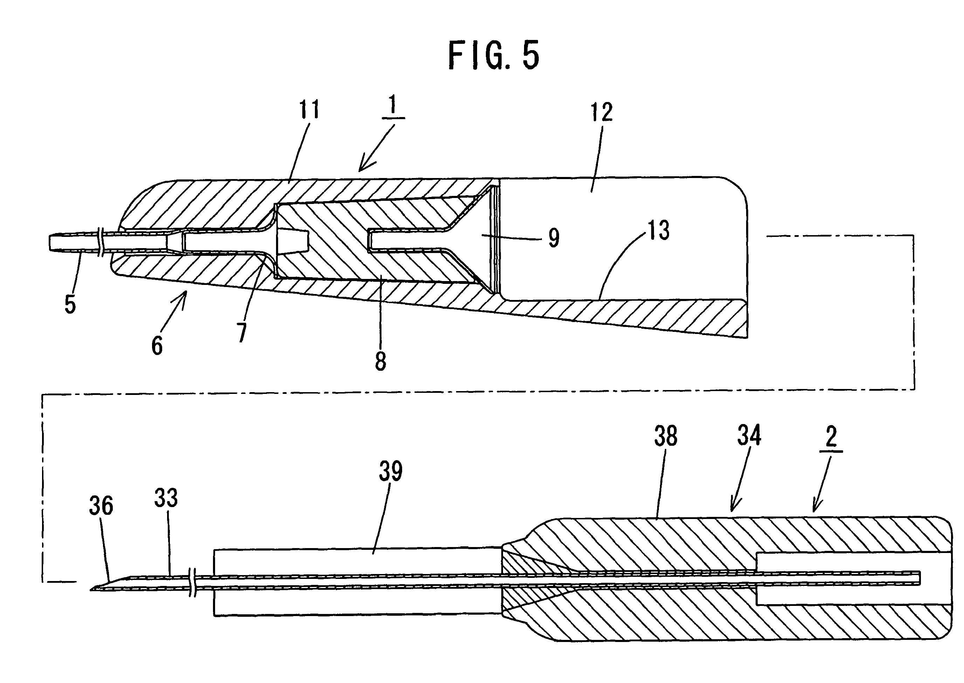

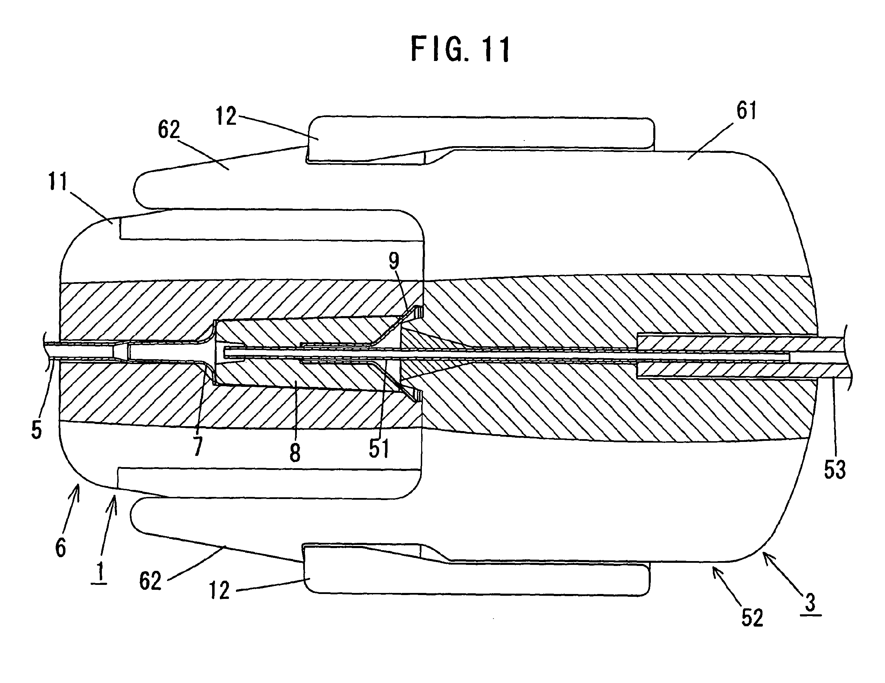

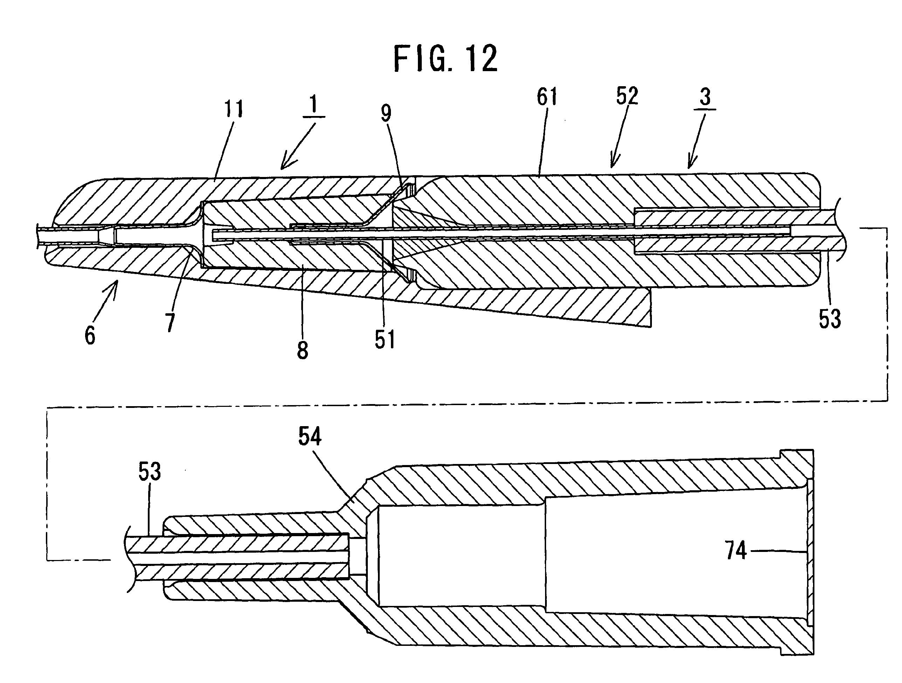

Referring now to Fig. 1 to Fig. 16, the first embodiment in which the present invention is applied to an insulin administration set will be described. As shown in Fig. 1 to Fig. 5, and Fig. 10 to Fig. 12, the administration set includes a cannula housing 1, an insertion hub 2, and an infusion hub 3.

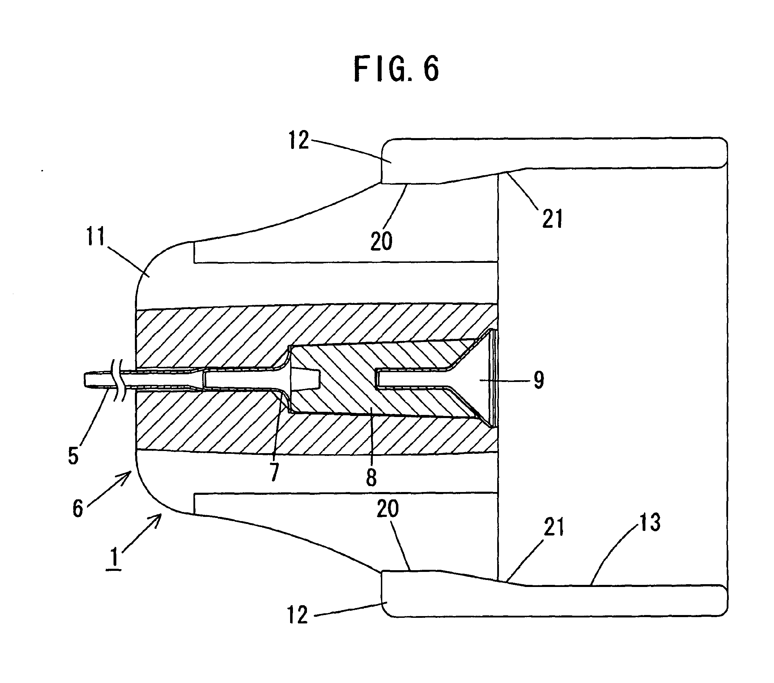

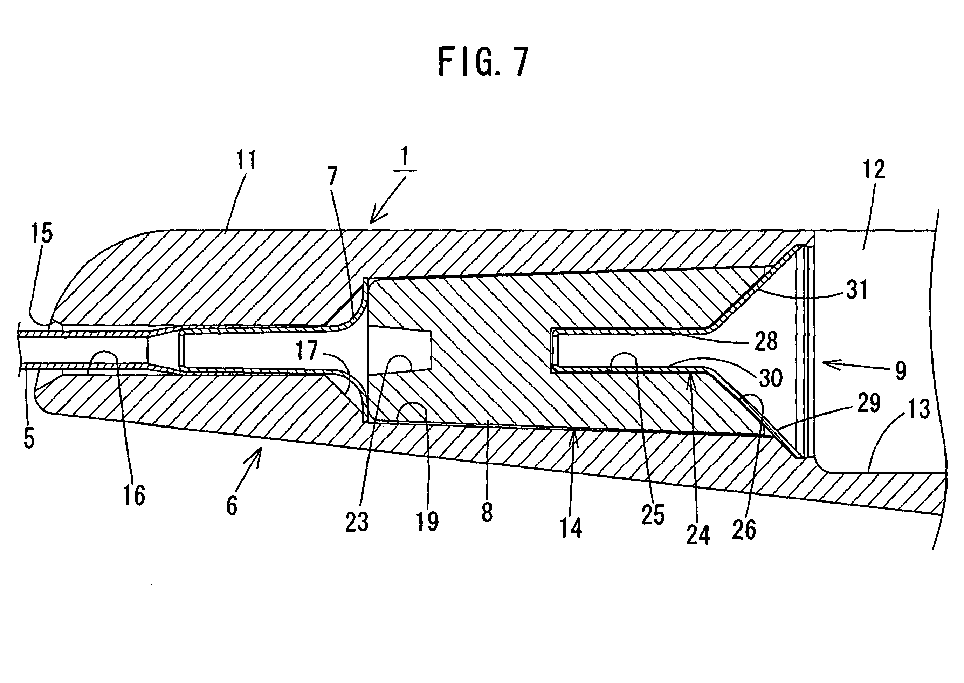

As shown also in Fig. 6 and Fig. 7, the cannula housing 1 includes a catheter (soft cannula, outer needle, shielding needle, catheter tube) 5, catheter hub (indwelling needle base, outer needle base, shielding needle base) 6, a fixing device (a caulking pin for fixation) 7, a plug 8, a guide (a caulking pin for guiding) 9.

The catheter 5 is disposed (substantially) in the axial direction, and formed into an elongated transparent (translucent) hollow (tubular) body having openings at its distal and proximal ends. The catheter 5 has flexibility and is formed integrally of plastic material (resin material). The plastic material includes, for example, thermoplastic resin. The thermoplastic resin used here is, preferably, polytetrafluoroethylene (PTFE), ethylene-tetrafluoroethylene copolymer (ETFE), polyurethane (PU), tetrafluoroethylene-perfluoroalkyl-vinyl-ether copolymer (PFA), polypropylene (PP), polyethylene (PE), polyvinyl chloride (PVC), acrylonitrile-butadiene-styrene copolymer, polycarbonate, polyamide, polyoxymethylene, and more preferably, PTFE, ETFE, PP, and PU.

The catheter hub 6 includes an upwardly projecting central protrusion (projection) 11 at the lateral center of the distal portion, upwardly projecting side protrusions (projections) 12 on the left and the right sides of the proximal portion, and a depression 13 depressed downward at the remaining portion. At the lateral center of the central protrusion 11, a through bore (insertion bore) 14 is formed so as to penetrate (substantially) in the axial direction. The through bore 14 includes a proximally tapered distal tapered bore 15, a straight bore 16 having a constant inner diameter in the direction of the axial center, a proximally tapered bore 17 tapered toward the distal end, and a connecting port 19 for feeding liquid having a larger diameter than the straight bore 16, all communicated with each other arranged in above-described sequence toward the proximal end. The proximal portion of the catheter 5 is inserted and fixed in the straight bore 16. The connecting port 19 may have a constant inner diameter in the direction of the axial center, or tapered toward the distal end at a small taper ratio depending on the cases. The inward side surfaces of the side protrusions 12 serve as guiding surfaces 20, and the axially central portions of the guiding surfaces 20 are beveled surfaces 21 extending inwardly as it proceeds to the distal end. The catheter hub 6 is integrally formed of plastic material (resin material), and the plastic material is preferably the same as those of the catheter 5, polyethersulfone (PES), or ABS resin, and more preferably, PP, PE, PES, PVC, and ABS resin.

The fixing device 7 clamps and fixes the proximal portion of the catheter 5 in cooperation with the inner surface of the straight bore 16 of the through bore 14, and is a hollow body having openings on the axial ends thereof. The portion other than the proximal end portion is a straight portion having a small outer diameter constant in the direction of axial center, and the proximal end portion is flared so as to be enlarged toward the proximal end. Fixation of the catheter 5 with respect to the catheter hub 6 is achieved in the following manner. In a first step, the heated fixing device 7 is press-fitted into the proximal portion of the catheter 5. At this time, the proximal portion of the catheter 5 is enlarged radially outwardly. Then, the catheter 5 and the fixing device 7 are inserted into the through bore 14 of the catheter hub 6 from its proximal end, and the proximal portion of the catheter 5 is clamped and fixed between the small diameter straight portion of the fixing device 7 and the inner surface of the straight bore 16 of the through bore 14. The fixing device 7 is integrally formed, for example, of stainless steel (SUS304 is preferable), nickel-titan alloy, plastic material (resin material).

The plug 8 is formed into substantially a columnar shape, and fitted and fixed into the connecting port 19 of the catheter hub 6 for sealing the connecting port 19. As an example of the above-described "fitted", the present invention employs "press-fitting", and hence the plug 8 is press-fitted into the connecting port 19. The term "press-fitting" means a state of "inserted in a state of being compressed in the axial and radial directions", and the reason to press-fit is for desirably sealing the connecting port 19 by bringing the outer surface of the plug 8 into press-contact (close contact) with the inner surface of the connecting port 19. However, even when the plug 8 is not press-fitted into the connecting port 19, as will be described later, the connecting port 19 is satisfactorily sealed by press-contact (close contact) of the plug 8 with the inner surface of the connecting port 19 by fitting (press-fitting and fixing) the guide 9 into a proximal bore 24 of the plug 8 and the connecting port 19 of the catheter hub 6. A distal bore 23 of truncated conical shape is punctuated from the center of the distal end surface of the plug 8 toward the proximal end, and the proximal bore (engaging bore) 24 is punctuated (formed) from the center of the proximal surface of the plug 8 toward the distal end. The proximal bore 24 includes a straight bore 25 formed on the distal side having a constant inner diameter in the direction of the axial center, and a tapered bore 26 is formed on the proximal side so as to be tapered toward the distal end. The plug 8 is formed of a resilient material, for example, rubber material such as isoprene rubber, silicon rubber, butyl rubber, thermoplastic elastomer, silicon elastomer, or latex.

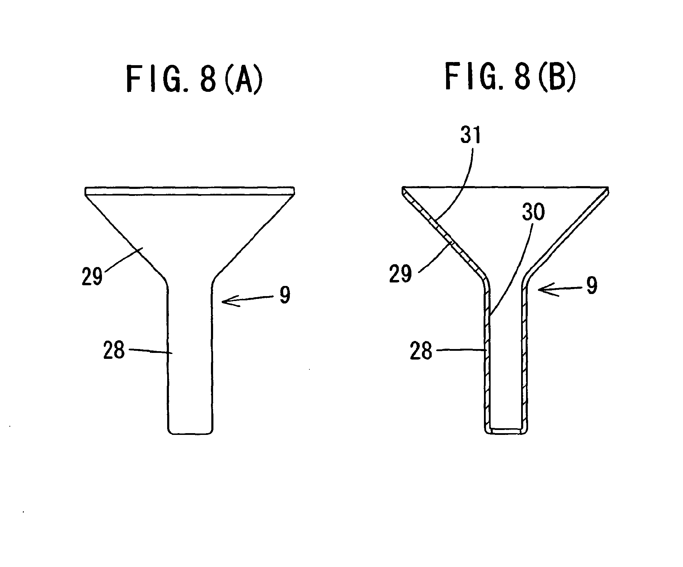

The guide 9 has both a function for retaining (fixing) the plug 8, and a function for guiding an insertion needle of an insertion hub 2 or an infusion needle of an infusion hub 3 described later, and is fitted (press-fitted and fixed) into the proximal bore 24 of the plug 8 and the connecting port 19 of the catheter hub 6. As shown also in Fig. 8, the guide 9 is formed into a hollow body having openings at its distal and proximal ends, and is integrally formed with a cylindrical portion 28 to be inserted (press-fitted) into the straight bore 25 of the proximal bore 24, the tapered bore 26 of the proximal bore 24, and a tapered portion 29 to be press-fitted into the proximal end of the connecting port 19. The guide 9 is fixed to the catheter hub 6 and hence the plug 8 is fixed to the catheter hub 6 by the proximal edge of the tapered portion 29 being press-fitted and fitted into the inner surface of the plug 8 while resiliently deforming the proximal end of the connecting port 19. Therefore, the plug 8 is prevented from falling off the connecting port 19. The interior of the cylindrical portion 28 is a straight bore 30 having a constant inner diameter in the direction of the axial center, and the interior of the tapered portion 29 is a distally tapered bore 31. The axial centers of the straight bore 30 and the tapered bore 31 (substantially) align with the axial center of the connecting port 19 of the cannula housing 1. The inner surface of the tapered bore 31 guides the insertion needle and the infusion needle to the (substantially) axial center of the plug 8 when they are inserted into the plug 8, which will be described below. The inner diameter of the distal end of the tapered bore 31, that is, the smallest inner diameter, and the inner diameter of the straight bore 30 are slightly larger than, but close to, the outer diameters of the insertion needle and the infusion needle as described later. The guide 9 is integrally formed of the same material as the fixing device 7.

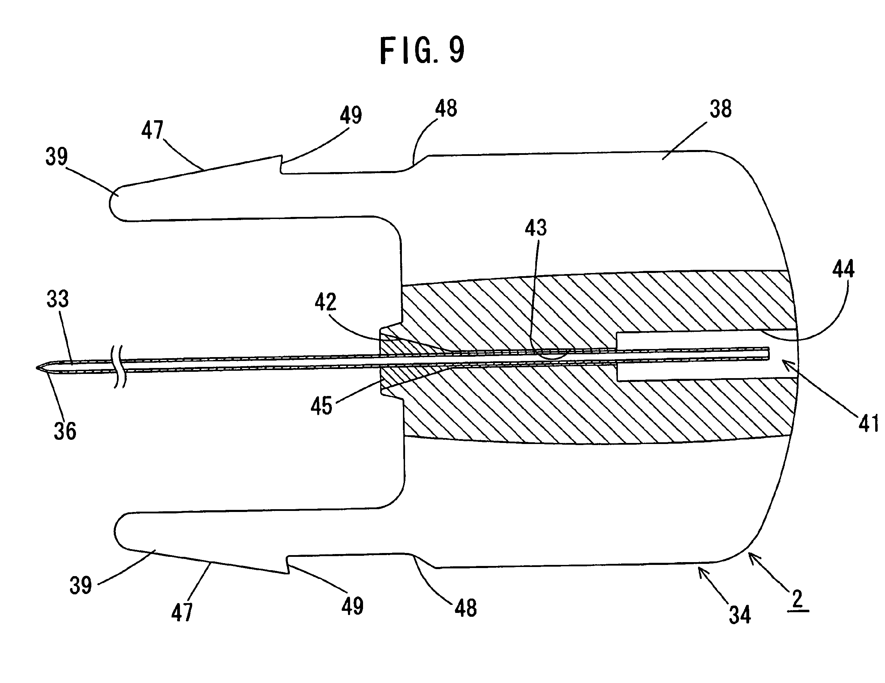

The insertion hub 2 is to be detachably connected to the cannula housing 1 from its proximal end and, as shown in Fig. 9, includes an insertion needle (inner needle) 33, and an insertion needle hub (inner needle base) 34.

The insertion needle 33 is detachably inserted into the guide 9, the plug 8, the fixing device 7, and the catheter 5 of the cannula housing 1 and projects distally from the catheter 5. The insertion needle 33 is disposed (substantially) in the axial direction, and is formed into a flexible elongated hollow (tubular) body having openings at its distal and proximal ends. The distal end of the insertion needle 33 is cut obliquely with respect to the axial center thereof (bevel cut) and hence a distal opening edge 36 is beveled to provide a sharp cutting edge. The distal opening of the insertion needle 33 faces upward. The insertion needle 33 is integrally formed, for example, of stainless steel (SUS 304 is preferable), or nickel-titan alloy.

The insertion needle hub 34 is provided at the proximal portion of the insertion needle 33, and is to be detachably connected to the catheter hub 6 of the cannula housing 1 from its proximal end, and includes integrally formed a main body 38 and a pair of left and right engaging claws 39. The insertion needle hub 34 is formed of the same material as the catheter hub 6.

An insertion bore 41 for the insertion needle is formed at the lateral center of the main body 38 so as to penetrate (substantially) in the axial direction. The insertion bore 41 includes a proximally tapered bore 42, a straight bore 43 having a constant inner diameter in the direction of axial center, a depressed portion 44 having an inner diameter constant in the direction of the axial center and larger than the outer diameter of the straight bore 43, all communicated with each other arranged in above-described sequence toward the distal end. The insertion needle 33 is inserted from its distal end into the insertion bore 41, and fixed to the inner surface of the tapered bore 42 and the straight bore 43 with an adhesive agent 45, so as to project distally from the insertion needle hub 34 to a large extent. By forming the tapered bore 42 in the insertion bore 41, the insertion needle 33 can be inserted easily into the insertion bore 41. There is a case that the insertion needle 33 is welded to the insertion needle hub 34. When connecting the insertion hub 2 to the cannula housing 1, the main body 38 is inserted from the proximal end of the depression 13 of the catheter hub 6, that is, behind the central protrusion 11 and between the side protrusions 12, so that the distal surface abuts against the proximal surface of the central protrusion 11.

The engaging claws 39 each constitute a locking mechanism for the insertion hub 2 in cooperation with the side protrusions 12 of the catheter hub 6, and project distally from the left and the right sides of the distal portion of the main body 38 so as to be capable of swinging in the lateral direction by resilient deformation of their own. The outward side surfaces of the distal portions of the engaging claws 39 are beveled surfaces 47 inclining inwardly as they proceed toward the distal end, and the proximal sides of the beveled surfaces 47 on the above-described outward side surfaces are formed with depressions 48 which are depressed inwardly, and the distal inner surfaces of the depressions 48 serve as engaging portions 49 having proximally facing planar surfaces, respectively. When connecting the insertion hub 2 into the cannula housing 1, the engaging claws 39, being guided by the guiding surface 20 of the catheter hub 6 and resiliently deformed inwardly, are inserted between the central protrusion 11 and the side protrusions 12 of the catheter hub 6. When the bevel surfaces 47 are moved distally of the side protrusions 12 of the catheter hub 6, they swing outwardly by their resilient recovery force, and the engaging portions 49 move distally of the side protrusions 12, so that an accidental disconnection of the insertion hub 2 from the cannula housing 1 is prevented. Disconnection of the insertion hub 2 from the cannula housing 1 can easily be done by holding both engaging claws 39 and swinging inwardly, moving the engaging portions 49 inwardly of the side protrusions 12 and moving the insertion hub 2 proximally of the cannula housing 1.

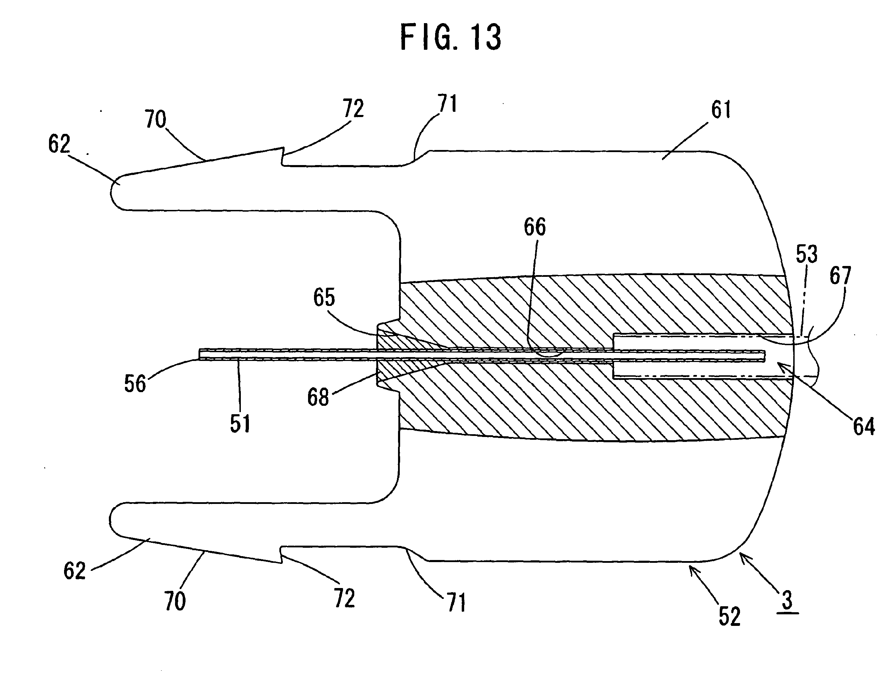

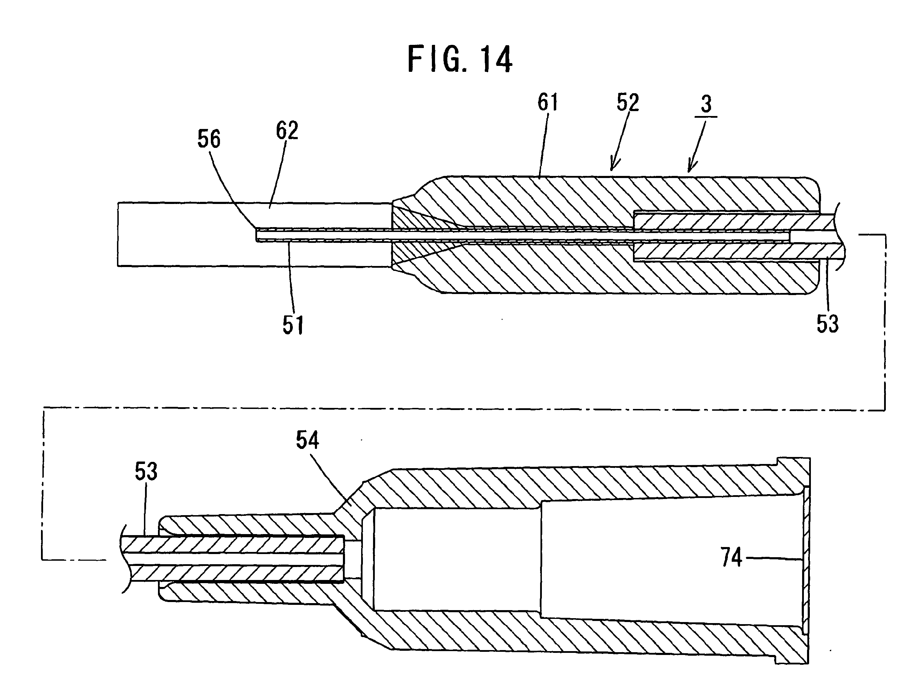

The infusion hub 3, which is to be detachably connected to the cannula housing 1 from its proximal end and, as shown in Fig. 13 and Fig. 14 as well, includes an infusion needle (solution infusion needle) 51 for feeding liquid, an infusion tubing hub (infusion needle base, solution infusion needle base) 52, a tube (liquid feeding line) 53, and a connector 54.

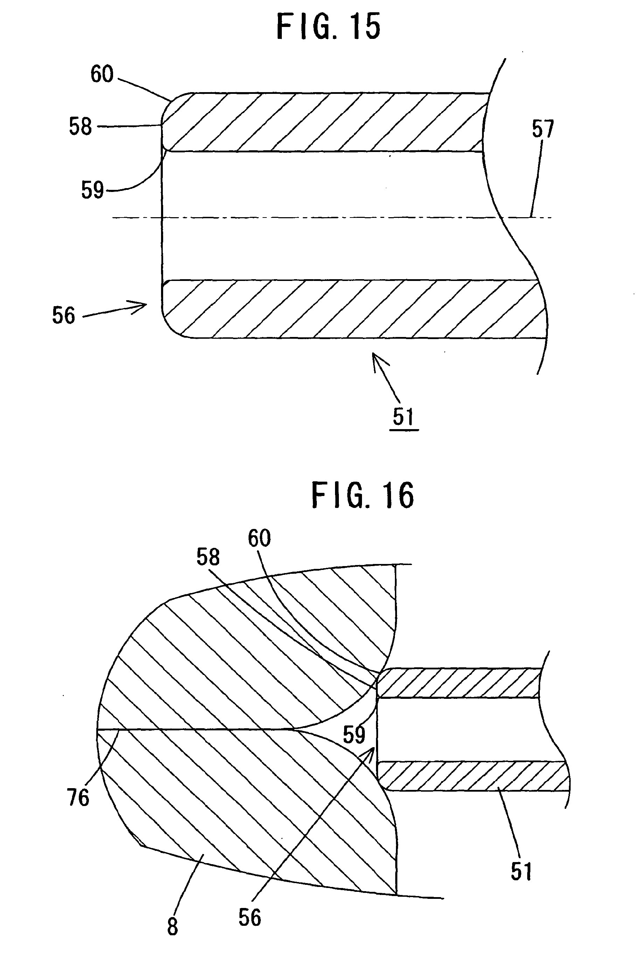

The infusion needle 51 is to be connected to the liquid feeding line and detachably inserted into the guide 9 and the plug 8 of the cannula housing 1 so as to communicate with the catheter 5. The infusion needle 51 is disposed (substantially) in the axial direction and formed into a flexible elongated hollow (tubular) body having openings at its distal and proximal ends, and has (substantially) the same outer diameter as the insertion needle 33. As shown in Fig. 15, a surface of an (entire) opening edge 56 of the distal end of the infusion needle 51 is orthogonal to an axial center 57 of the infusion needle 51. The opening edge 56 (The surface of the opening edge 56) of the infusion needle 51 is smoothly chamfered, and at least (the surface (outer surface) of) the outer peripheral side (periphery) and the inner peripheral side of the opening edge 56 is formed into an outwardly projecting (swelling) curved surface (curved projection) so as to make the opening edge 56 into a nonsharp state. Therefore, the infusion needle 51 has little or no puncturing capability. More specifically, in the present example, (the surface (outer surface) of) the opening edge 56 has a flat surface (plane face) 58 having a distally facing planar surface at the radially center thereof, an inwardly projecting (swelling) curved surface (curved face, curved projection) 59 curvedly projecting (swelling) distally and radially inwardly at the inner peripheral side (periphery) thereof, and an outwardly projecting (swelling) curved surface (curved face, curved projection) 60 curvedly projecting (swelling) distally and radially outwardly at the outer peripheral side (periphery) thereof. It is also possible to form the entire opening edge 56 into an outwardly (distally) projecting (swelling) curved surface (curved projection) without providing a flat surface. The infusion needle 51 is integrally formed, for example, of stainless steel (SUS 304 is preferable). The infusion needle 51 is coated with lubricant such as silicone oil or the like applied thereon.

An infusion tubing hub 52, being provided at the proximal portion of the infusion needle 51, is to be detachably connected to the catheter hub 6 of the cannula housing 1 from its proximal end, and includes a main body 61 and a pair of left and right engaging claws 62 integrally formed with each other, similar to the insertion needle hub 34. The infusion tubing hub 52 is formed of the same material as the catheter hub 6 or the insertion needle hub 34.

An infusion needle insertion bore 64 is formed (substantially) in the axial direction so as to penetrate through the lateral center of the main body 61. The insertion bore 64 includes a proximally tapered bore 65, a straight bore 66 having a constant inner diameter in the direction of the axial center, a tube fitting bore 67 having an inner diameter constant in the direction of the axial center and larger than that of the straight bore 66, all communicated with each other arranged in the above-described sequence toward the proximal end. The infusion needle 51 is inserted into the insertion bore 64 from its distal end, and fixed to the inner surfaces of the tapered bore 65 and the straight bore 66 with an adhesive agent 68 so as to project distally from the main body 61. By forming the tapered bore 65 in the insertion bore 64, the infusion needle 51 can easily be inserted into the insertion bore 64. There is also a case in which the infusion needle 51 is welded to the infusion tubing hub 52. When connecting the infusion hub 3 to the cannula housing 1, the main body 61 is inserted into the proximal portion of the depression 13 of the catheter hub 6, that is, behind the central protrusion 11 and between the side protrusions 12 from its proximal end, so that the distal surface abuts against the proximal surface of the central protrusion 11.

The engaging claws 62 each constitute a locking mechanism for the insertion hub 3 in cooperation with the side protrusions 12 of the catheter hub 6, and project distally from the left and the right sides of the distal portion of the main body 61 so as to be capable of swinging in the lateral direction by resilient deformation of their own. The outward side surfaces of the distal portions of the engaging claws 62 are beveled surfaces 70 inclining inwardly as they proceed toward the distal end, and the proximal sides of the beveled surfaces 70 on the above-described outward side surface are formed with depressions 71 which are depressed inwardly, and the distal inner surfaces of the depressions 71 serve as engaging portions 72 having proximally facing planar surfaces, respectively. When connecting the infusion hub 3 to the cannula housing 1, the engaging claws 62, being guided by the guiding surface 20 of the catheter hub 6 and resiliently deformed inwardly, are inserted between the central protrusion 11 and the side protrusions 12 of the catheter hub 6. When the beveled surfaces 70 are moved distally of the side protrusions 12 of the catheter hub 6, they swing outwardly by their resilient recovery force, and the engaging portions 72 move distally of the side protrusions 12, so that an accidental disconnection of the infusion hub 3 from the cannula housing 1 is prevented. Disconnection of the infusion hub 3 from the cannula housing 1 can easily be done by holding both engaging claws 62 and swinging inwardly, moving the engaging portions 72 inwardly of the side protrusions 12 and moving the infusion hub 3 proximally of the cannula housing 1.

The tube 53 is an example of the liquid feeding line, and is transparent (translucent). The distal end of the tube 53 is fitted on the proximal portion of the infusion needle 51, and is fixed to the inner surface of the tube fitting bore 67 of the insertion bore 64 of the infusion tubing hub 52, for example, with adhesion or welding. The length of the tube 53 may be freely determined as needed depending on the location of the insulin administration pump (not shown). The tube 53 is integrally formed of plastic material.

The connector 54 is a hollow body having openings at its distal and proximal ends, and fitted onto the proximal end of the tube 53 in communication with each other, and is to be connected to an insulin administration pump directly or indirectly via a connecting line (member) such as a tube. The opening of the proximal end of the connector 54 is sealed by a sealing member 74 such as a filter or a lid member before connecting the connecting line to the connector 54. The connector 54 is integrally formed of the same material as the catheter hub 6.

In the first embodiment described above, insertion of the catheter 5 under the skin of a patient is carried out by connecting the insertion hub 2 to the cannula housing 1, connecting the insertion needle hub 34 of the insertion hub 2 to the catheter hub 6 of the cannula housing 1 from its proximal end, and inserting the insertion needle 33 of the insertion hub 2 to the guide 9, the plug 8, the fixing device 7, and the catheter 5 of the cannula housing 1 from its proximal end as shown in Fig. 1 to Fig. 3, and when inserting the insertion needle 33 into the plug 8, the plug 8 is resiliently deformed.

The axial centers of the straight bore 25 and the tapered bore 26 of the guide 9 are (substantially) aligned with the axial center of the connecting port 19 of the cannula housing 1, and the inner diameter of the distal end of the tapered bore 26, that is, the smallest inner diameter, and the inner diameter of the straight bore 25 are slightly larger than the outer diameter of the insertion needle 33. Therefore, when inserting the insertion needle 33 as described above, the sharp edged distal end of the insertion needle 33 is guided by the inner surface of the tapered bore 26 of the guide 9 while being centered, and is guided into the (substantially) axial center of the guide 9, that is, of the connecting port 19 so as to be inserted easily into the straight bore 25 of the guide 9. Accordingly, the insertion needle 33 can easily be inserted into the (substantially) axial center of the plug 8, and the insertion needle 33 is projected distally from the distal bore 23 of the plug 8, so as to be inserted into the catheter 5 of via the fixing device 7.

In this manner, in a state in which the insertion hub 2 is connected to the cannula housing 1, the insertion needle 33 and the catheter 5 are inserted under the skin of the patient. Then, the insertion hub 2 is disconnected from the cannula housing 1 to pull out the insertion needle 33 from under the skin of the patient and from the cannula housing 1, and the catheter 5 is indwelled under the skin of the patient. In this case, the trace (puncture) formed by the insertion needle 33 on the plug 8 is sealed by resilient recovering force of the plug 8. Therefore, the risk of leakage of blood from the connecting port 19 of the cannula housing 1 or entrance of outside air from the connecting port 19 into the through bore 14 may be eliminated.

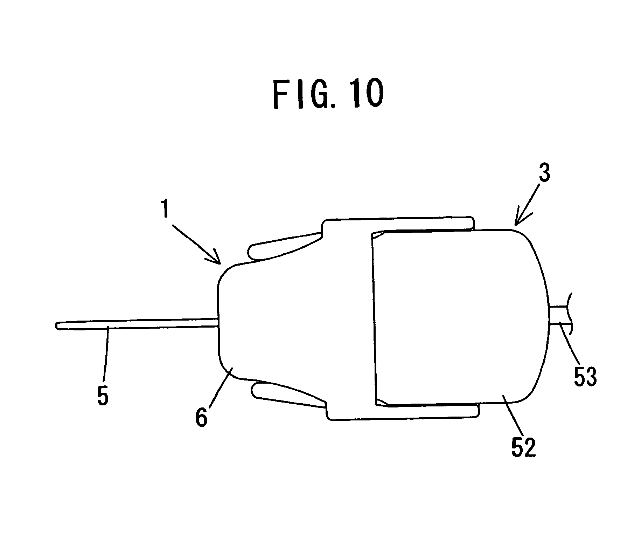

Subsequently, discontinuous or continuous administration of insulin is carried out, as shown in Fig. 10 to Fig. 12, by connecting the infusion hub 3, to which the insulin administration pump is connected, to the cannula housing 1, connecting the infusion tubing hub 52 of the infusion hub 3 to the catheter hub 6 of the cannula housing 1 from its proximal end, and then inserting the infusion needle 51 of the infusion hub 3 to the guide 9 and the plug 8 of the cannula housing 1 from its proximal end so as to communicate with the catheter 5. The distal opening edge 56 has a flat surface 58 having a distally facing planar surface at the radially center thereof, an inwardly projecting (swelling) curved surface 59 curvedly projecting distally and radially inwardly at (the surface of) the inner peripheral side (periphery) thereof, and an outwardly projecting (swelling) curved surface 60 curvedly projecting (swelling) distally and radially outwardly at (the surface of) the outer peripheral side (periphery) thereof. Therefore, the infusion needle 51 itself has little or no puncturing capability.

However, since the trace of the insertion needle 33, which is pulled out, exists in the closed state at the (substantially) axial center of the plug 8, the infusion needle 51 is inserted into the trace 76 of the plug 8 so as to follow the trace 76 while resiliently deforming the plug 8 as shown in Fig. 16.

In this manner, when the infusion needle 51 is inserted into the plug 8, the infusion needle 51 neither tears the plug 8 while advancing, nor cuts off the plug 8 by the inner peripheral side (periphery) of the opening edge 56 of the infusion needle 51 as in the related art, but the infusion needle 51 is inserted into the trace 76 of the insertion needle 33.

Therefore, even when insertion of the infusion needle 51 into the plug 8 is repeated for administrating insulin depending on the condition of the patient, the possibility of occurrence of a phenomenon that a bore is formed in the plug 8 (coring) because the plug 8 is ground off is extremely low. Therefore, the risk of deterioration of the sealing performance of the connecting port 19 by the plug 8 is extremely low, and the risk of entering of the "ground chips" of the plug 8 into the infusion needle 51 or the catheter 5, and eventually into the patient's body is extremely low.

In addition, as described above, the infusion needle 51 itself has little or no capability of puncturing the plug 8, and the infusion needle 51 is inserted into the trace 76 of the insertion needle 33. Therefore, the risk that the infusion needle 51 is inserted into the plug 8 at different positions at every insertion into the plug 8 and hence tears the plug 8 is extremely low. Therefore, even when insertion of the infusion needle 51 into the plug 8 is repeated, the risk that the plug 8 loses its original strength or the recovering performance is extremely low, and hence the risk that the sealing performance of the plug 8 with respect to the connecting port 19 is deteriorated is extremely low and the risk that the plug 8 is repeatedly torn is also extremely low as described above. Consequently, the risk of generating fine strips of the plug 8 is extremely low, and the risk that the fine strips of the plug 8 enter into the patient's body is also extremely low.

When the guide 9 is not provided on the cannula housing 1, the position of the plug 8 where the insertion needle 33 is to be inserted does not align with the (substantially) axial center of the plug 8, but is dispersed in a wide area including the axial center of the plug 8, and the infusion needle 51 can hardly follow the trace 76 of the insertion needle 33 in the plug 8.

However, according to the present embodiment, the guide 9 is provided on the cannula housing 1, the axial centers of the straight bore 30 and the tapered bore 31 of the guide 9 (substantially) align with the axial center of the connecting port 19 of the cannula housing 1, and the inner diameter at the distal end of the tapered bore 31, that is, the smallest inner diameter thereof, and the inner diameter of the straight bore 30 are slightly larger than the outer diameter of the insertion needle 33. Therefore, as described above, the insertion needle 33 is inserted into the (substantially) axial center of the plug 8. When the infusion needle 51 is inserted into the plug 8 as well, the infusion needle 51 is guided by the inner surface of the tapered bore 31 of the guide 9 while being centered, and is guided into the (substantially) axial center of the guide 9, that is, of the connecting port 19 so as to be easily inserted into the straight bore 30 of the guide 9. In addition, the infusion needle 51 has (substantially) the same outer diameter as the insertion needle 33, and the outer diameter thereof is slightly smaller than the inner diameter of the straight bore 30. Therefore, the infusion needle 51 can easily be inserted into the trace 76 in the plug 8 smoothly by following (tracing) the trace 76 of the insertion needle 33, and insertion and pulling out of the infusion needle 51 into/from the trace 76 can easily be repeated.

In addition, as described above, since the infusion needle 51 itself has little or no puncturing capability, the risk that the user picks his/her finger or hand is extremely low. Therefore, it is not necessary to provide a protective wall for surrounding the infusion needle 51 as shown in USP No.6056718, and hence the infusion hub 3 can be downsized. In addition, since the infusion needle 51 is coated with lubricant applied thereon, the infusion needle 51 can be inserted into the trace 76 in the plug 8 easily and smoothly.

By driving the insulin administration pump after the infusion needle 51 of the infusion hub 3 is inserted into the plug 8 of the cannula housing 1 as described above, the insulin can be administered into the patient's body discontinuously or continuously via the connector 54, the tube 53, the infusion needle 51, the fixing device 7, and the catheter 5 of the infusion hub 3 from the pump.

When the administration is completed, the infusion hub 3 is disconnected from the cannula housing 1 to disconnect the infusion tubing hub 52 of the infusion hub 3 from the catheter hub 6 of the cannula housing 1, and then the infusion needle 51 of the infusion hub 3 is pulled out from the plug 8 and the guide 9 of the cannula housing 1. In this case, the trace of the infusion needle 51 (that is, the trace 76 of the insertion needle 33) of the plug 8 is closed by the resilient recovering force of the plug 8, and the risk of leakage of blood from the connecting port 19 of the cannula housing 1 or entrance of outside air from the connecting port 19 into the through bore 14 is eliminated.

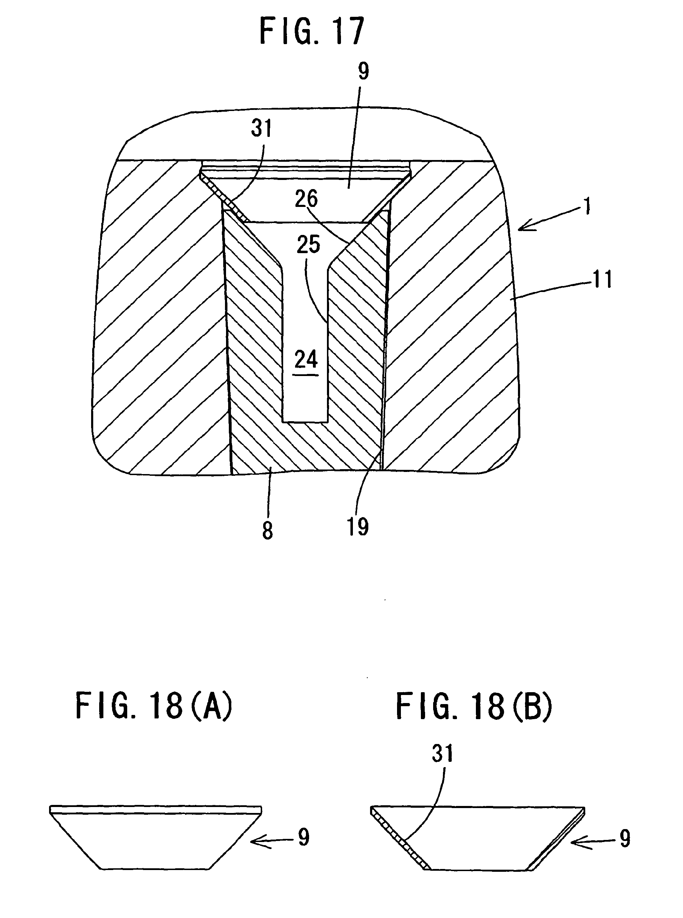

Fig. 17 and Fig. 18 show a second embodiment of the present invention, in which the guide 9 has a configuration as in the first embodiment but having the cylindrical portion 28 removed from the guide 9 and being tapered toward the distal end.

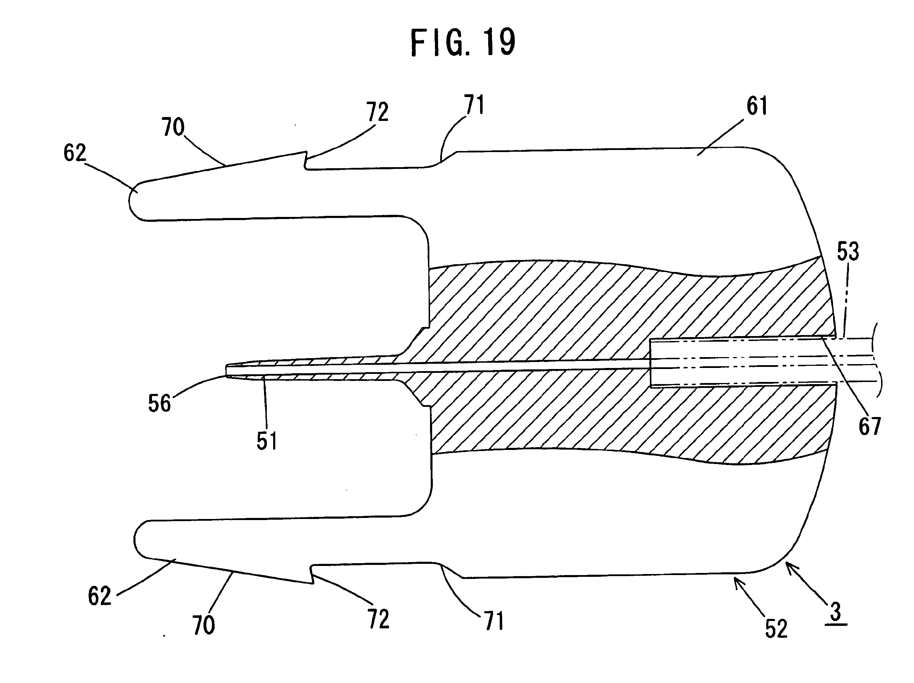

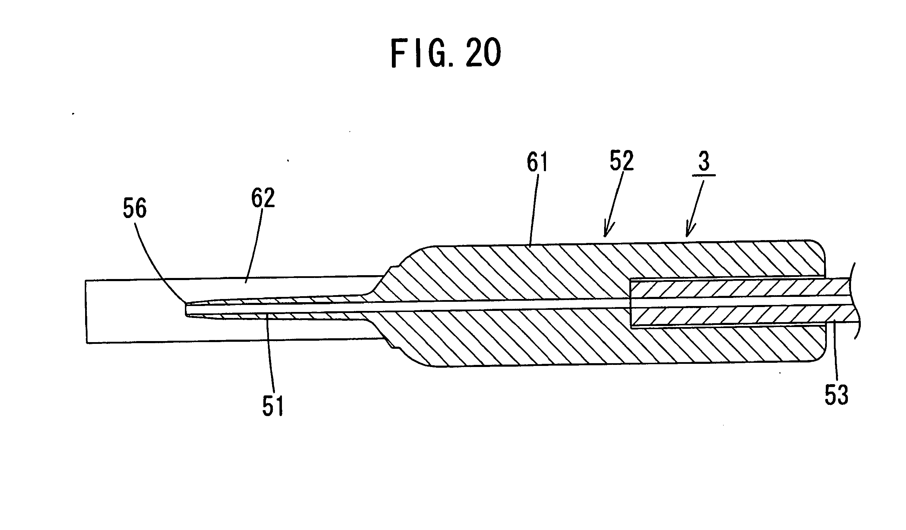

Fig. 19 and Fig. 20 show a third embodiment of the present invention, and the infusion needle 51 and the infusion tubing hub 52 of the infusion hub 3 are integrally molded of plastic material. At the time of molding, the opening edge 56 of the distal end of the infusion needle 51 is molded as in the same manner as the first embodiment.

In the first embodiment, it is necessary to carry out machining to the opening edge 56 of the distal end of the infusion needle 51, and hence the number of machining steps increases correspondingly in comparison with the related art, and hence increase in manufacturing cost may result. Also, in the first embodiment, the step of adhering or welding the infusion needle 51 to the infusion hub 52 is also necessary as in the related art.

However, according to the third embodiment, the infusion needle 51 and the infusion tubing hub 52 are integrally molded, and at the time of molding, the opening edge 56 of the distal end of the infusion needle 51 is molded as in the first embodiment. Therefore, it is not necessary to carry out machining to the opening edge 56 of the distal end of the infusion needle 51, and hence the number of machining steps does not increase in comparison with the related art. In addition, the process of adhering or welding the infusion needle 51 to the infusion tubing hub 52, which has been necessary in the related art as well, is not necessary. Therefore, according to the third embodiment, the cost of manufacturing the infusion hub 3, that is, the insulin administration set may be reduced with respect to the related art.

In the embodiments described above, the present invention is applied to an insulin administration set. However, the present invention is applicable to (1) various indwelling catheter sets for solution infusion other than insulin, blood transfusion, blood collection, dialysis therapy, and so on, and (2) various connectors for medical use in which the cannula housing does not have a catheter, and is provided on a solution infusion line, a blood transfusion line, a blood collection line, and an arterial and venous line for dialysis therapy.

## Claims

1. A connector set for medical use comprising:
(a) a cannula housing (1); and
(b) an infusion hub (3) to be detachably connected to the cannula housing (1) from its proximal end,
the cannula housing (1) comprising:
(a) a catheter hub (6), a proximal portion of which is formed into a connecting port (19) for feeding liquid; and
(b) a plug (8) formed of resilient material for sealing the connecting port (19), the plug (8) being inserted into the connecting port (19),
the infusion hub (3) comprising:
(a) an infusion needle (51) for feeding liquid disposed substantially in the axial direction and formed into a hollow body opened at its distal and proximal ends, the infusion needle (51) being detachably inserted into the plug (8) from its proximal end when connecting the infusion hub (3) and the cannula housing (1); and
(b) an infusion tubing hub (52) provided at a proximal portion the infusion needle (51), the infusion tubing hub (52) being detachably connected to the catheter hub (6) from its proximal end when connecting the infusion hub (3) and the cannula housing (1),
**characterized in that**
an opening edge (56) of distal end of the infusion needle (51) is orthogonal to an axial center (57) of the infusion needle (51),
an inner peripheral side and an outer peripheral side of the opening edge (56) being formed into outwardly projecting curved surfaces (59, 60).

2. A connector set for medical use according to claim 1, further comprising:
an insertion hub (2) to be detachably connected to the cannula housing (1) from its proximal end,
the insertion hub (2) comprising:
(a) an insertion needle (33) disposed substantially in the axial direction, the insertion needle (33) being detachably inserted into the plug (8) from its proximal end when connecting the insertion hub (2) and the cannula housing (1); and
(b) an insertion needle hub (34) provided at a proximal portion of the insertion needle (33), the insertion needle hub (34) being detachably connected to the catheter hub (6) from its proximal end when connecting the insertion hub (2) and the cannula housing (1).

3. A connector set for medical use according to claim 1 or 2, further comprising:
a guide (9) formed into a hollow body opened at its distal and proximal ends for preventing dropping off of the plug (8) from the connecting port (19),
the guide (9) being inserted into a portion of the connecting port (19), which is disposed proximally of the plug (8),
the guide (9) allowing detachable insertion of the infusion needle (51) from its proximal end,
the guide (9) being formed with a tapered bore (31) having a distally tapered shape at least at a proximal portion in the interior of the guide (9),
an inner surface of the tapered bore (31) being configured to guide the infusion needle (51) toward a substantially axial center of the connecting port (19).

4. A connector set for medical use according to claim 3, wherein the guide (9) comprises in the interior:
(a) a straight bore (30) forming a distal portion of the interior of the guide (9) and having an inner diameter substantially constant in the axial direction; and
(b) the tapered bore (31) forming the proximal portion of the interior of the guide (9).

5. A connector set for medical use according to any one of claims 1-4, wherein the infusion needle (51) and the infusion tubing hub (52) are integrally formed of plastic material,
the opening edge (56) of the distal end of the infusion needle (51) being formed to be orthogonal to an axial center (57) of the infusion needle (51)and the inner peripheral side and the outer peripheral side of the opening edge (56) being formed into the outwardly projecting curved surfaces (59, 60) at the time of molding.

6. A connector set for medical use according to any one of claims 1-5, wherein the infusion needle (51) is coated with lubricant applied thereon.

7. An indwelling catheter set comprising:
the connector set according to any one of claims 1-6,
wherein a through bore (14) is formed in the catheter hub (6) of the cannula housing (1) substantially in the axial direction,
a proximal portion of the through bore (14) being formed into the connecting port,
a catheter (5) being inserted into a distal portion of the through bore (14),
the catheter (5) being disposed substantially in the axial direction, and being formed into a hollow body opened at its distal and proximal ends,
the catheter (5) being communicated with the infusion needle (51) when the infusion needle (51) is inserted into the plug (8).

## Patentansprüche

1. Verbindersatz zur medizinischen Verwendung, umfassend:
(a) ein Kanülengehäuse (1); und
(b) einen Infusionsansatz (3), welcher mit dem Kanülengehäuse (1) von dessem proximalen Ende lösbar zu verbinden ist,
wobei das Kanülengehäuse (1) umfasst:
(a) einen Katheteransatz (6), wobei ein proximaler Abschnitt des Katheteransatzes zu einer Verbindungsöffnung (19) zum Zuführen von Flüssigkeit ausgebildet ist; und
(b) einen Stopfen (8) zum Abdichten der Verbindungsöffnung (19), welcher aus einem elastischen Material ausgebildet ist,
wobei der Stopfen (8) in die Verbindungsöffnung (19) eingeführt ist,
wobei der Infusionsansatz (3) umfasst:
(a) eine Infusionsnadel (51) zum Zuführen von Flüssigkeit, welche im Wesentlichen in der Axialrichtung angeordnet und zu einem an seinen distalen und proximalen Enden geöffneten Hohlkörper ausgebildet ist, wobei die Infusionsnadel (51) beim Verbinden des Infusionsansatzes (3) und des Kanülengehäuses (1) in den Stopfen (8) von dessem proximalen Ende lösbar eingeführt wird; und
(b) einen Infusionsschlauchansatz (52), welcher an einem proximalen Abschnitt der Infusionsnadel (51) vorgesehen ist, wobei der Infusionsschlauchansatz (52) beim Verbinden des Infusionsansatzes (3) und des Kanülengehäuses (1) mit dem Katheteransatz (6) von dessem proximalen Ende lösbar verbunden wird,
**dadurch gekennzeichnet,**
**dass** ein Öffnungsrand (56) eines distalen Endes der Infusionsnadel (51) orthogonal zu einer axialen Mitte (57) der Infusionsnadel (51) ist,
wobei eine Innenrandseite und eine Außenrandseite des Öffnungsrands (56) zu nach außen hervorragenden gekrümmten Flächen (59, 60) ausgebildet sind.

2. Verbindersatz zur medizinischen Verwendung nach Anspruch 1, weiterhin umfassend:
einen Einführansatz (2), welcher mit dem Kanülengehäuse (1) von dessem proximalen Ende lösbar zu verbinden ist,
wobei der Einführansatz (2) umfasst:
(a) eine Einführnadel (33), welche im Wesentlichen in der Axialrichtung angeordnet ist, wobei die Einführnadel (33) beim Verbinden des Einführansatzes (2) und des Kanülengehäuses (1) in den Stopfen (8) von dessem proximalen Ende lösbar eingeführt wird; und
(b) einen Einführnadelansatz (34), welcher an einem proximalen Abschnitt der Einführnadel (33) vorgesehen ist, wobei der Einführnadelansatz (34) beim Verbinden des Einführansatzes (2) und des Kanülengehäuses (1) mit dem Katheteransatz (6) von dessem proximalen Ende lösbar verbunden wird.

3. Verbindersatz zur medizinischen Verwendung nach Anspruch 1 oder 2, weiterhin umfassend:
eine Führungseinrichtung (9), welche zu einem an seinen distalen und proximalen Enden geöffneten Hohlkörper ausgebildet ist, um ein Abfallen des Stopfens (8) von der Verbindungsöffnung (19) zu verhindern,
wobei die Führungseinrichtung (9) in einen Abschnitt der Verbindungsöffnung (19) eingeführt ist, welcher proximal zu dem Stopfen (8) angeordnet ist,
wobei die Führungseinrichtung (9) ein lösbares Einführen der Infusionsnadel (51) von ihrem proximalen Ende ermöglicht,
wobei die Führungseinrichtung (9) mit einer verjüngten Bohrung (31) ausgebildet ist, welche wenigstens an einem proximalen Abschnitt in dem Inneren der Führungseinrichtung (9) eine distal verjüngte Form aufweist,
wobei eine Innenoberfläche der verjüngten Bohrung (31) eingerichtet ist, um die Infusionsnadel (51) in Richtung einer im Wesentlichen axialen Mitte der Verbindungsöffnung (19) zu führen.

4. Verbindersatz zur medizinischen Verwendung nach Anspruch 3, wobei die Führungseinrichtung (9) in dem Inneren umfasst:
(a) eine gerade Bohrung (30), welche einen distalen Abschnitt des Inneren der Führungseinrichtung (9) ausbildet und einen in der Axialrichtung im Wesentlichen konstanten Innendurchmesser aufweist; und
(b) die verjüngte Bohrung (31), welche den proximalen Abschnitt des Inneren der Führungseinrichtung (9) ausbildet.

5. Verbindersatz zur medizinischen Verwendung nach einem der Ansprüche 1-4, wobei die Infusionsnadel (51) und der Infusionsschlauchansatz (52) aus einem Kunststoffmaterial integriert ausgebildet sind,
wobei der Öffnungsrand (56) des distalen Endes der Infusionsnadel (51) so ausgebildet ist, dass er orthogonal zu einer axialen Mitte (57) der Infusionsnadel (51) ist, und wobei die Innenrandseite und die Außenrandseite des Öffnungsrands (56) beim Formen zu den nach außen hervorragenden gekrümmten Flächen (59, 60) ausgebildet werden.

6. Verbindersatz zur medizinischen Verwendung nach einem der Ansprüche 1-5, wobei die Infusionsnadel (51) mit einem darauf aufgebrachten Schmiermittel beschichtet ist.

7. Verweilkathetersatz, umfassend:
den Verbindersatz nach einem der Ansprüche 1-6,
wobei eine Durchgangsbohrung (14) in dem Katheteransatz (6) des Kanülengehäuses (1) im Wesentlichen in der Axialrichtung ausgebildet ist,
wobei ein proximaler Abschnitt der Durchgangsbohrung (14) zu der Verbindungsöffnung ausgebildet ist,
wobei ein Katheter (5) in einen distalen Abschnitt der Durchgangsbohrung (14) eingeführt ist,
wobei der Katheter (5) im Wesentlichen in der Axialrichtung angeordnet ist und zu einem an seinen distalen und proximalen Enden geöffneten Hohlkörper ausgebildet ist,
wobei eine Verbindung zwischen dem Katheter (5) und der Infusionsnadel (51) hergestellt wird, wenn die Infusionsnadel (51) in den Stopfen (8) eingeführt wird.

## Revendications

1. Connecteur à usage médical comprenant :
(a) un corps de canule (1) ; et
(b) un raccord d'infusion (3) pouvant être relié de manière amovible au corps de canule (1) à son extrémité proximale;
le corps de canule (1) comprenant :
(a) un raccord de cathéter (6), dont une partie proximale est formée en un orifice de connexion (19) afin d'introduire un liquide ; et
(b) un bouchon (8) en matériau élastique destiné à obturer l'orifice de connexion (19), le bouchon (8) étant inséré dans l'orifice de connexion (19)
le raccord d'infusion (3) comprenant :
(a) une aiguille d'infusion (51) pour introduire un liquide disposée sensiblement dans la direction axiale et formée en un corps creux ouvert à ses extrémités distale et proximale, l'aiguille d'infusion (51) étant insérée de manière amovible dans le bouchon (8) à son extrémité proximale lorsqu'on connecte le raccord d'infusion (3) et le corps de canule (1) ; et
(b) un raccord de tubulure d'infusion (52) prévu à une extrémité proximale de l'aiguille d'infusion (51), le raccord de tubulure d'infusion (52) étant relié de manière amovible au raccord de cathéter (6) à son extrémité proximale une fois que le raccord d'infusion (3) et le corps de canule (1) ont été reliés mutuellement,
**caractérisé en ce**
**qu'**un bord d'ouverture (56) de l'extrémité distale de l'aiguille d'infusion (51) est orthogonal à un centre axial (57) de l'aiguille d'infusion (51) ;
**qu'**un côté périphérique intérieur et qu'un côté périphérique extérieur du bord d'ouverture (56) sont sous la forme respectivement de surfaces arrondies (59, 60) s'étendant vers l'extérieur.

2. Connecteur à usage médical selon la revendication 1 comprenant en outre :
un raccord d'insertion (2) relié de manière amovible au corps de canule (1) à son extrémité proximale,
le raccord d'insertion (2) comprenant :
(a) une aiguille d'insertion (33) disposée sensiblement dans la direction axiale, l'aiguille d'insertion (33) étant insérée de manière amovible dans le bouchon (8) à son extrémité proximale une fois que le raccord d'insertion (2) et le corps de canule (1) ont été reliés mutuellement ; et
(b) un raccord d'aiguille d'insertion (34) prévu à une extrémité proximale de l'aiguille d'insertion (33), le raccord d'aiguille d'insertion (34) étant raccordé de manière amovible au raccord de cathéter (6) à son extrémité proximale une fois que le raccord d'insertion (2) et le corps de canule (1) ont été reliés mutuellement.

3. Connecteur à usage médical selon la revendication 1 ou 2, comprenant en outre :
un guide (9) formé en un corps creux ouvert à ses extrémités distale et proximale afin d'empêcher la chute du bouchon (8) de l'orifice de connexion (19);
le guide (9) étant inséré dans une partie de l'orifice de connexion (19), agencée en position proximale par rapport au bouchon (8);
le guide (9) permettant une insertion de manière amovible de l'aiguille d'infusion (51) à son extrémité proximale ;
le guide (9) étant configuré de sorte à comporter un passage conique (31) avec conicité dans la direction distale au moins dans une partie proximale à l'intérieur du guide (9) ;
une surface intérieure du passage conique (31) étant configurée de sorte à guider l'aiguille d'infusion (51) vers un centre sensiblement axial de l'orifice de connexion (19).

4. Connecteur à usage médical selon la revendication 3, dans lequel le guide (9) comprend en partie intérieure :
(a) un passage droit (30) formant une partie distale de l'intérieur du guide (9) et de diamètre intérieur sensiblement constant dans la direction axiale ; et
(b) le passage conique (31) formant la partie proximale de l'intérieur du guide (9).

5. Connecteur à usage médical selon l'une quelconque des revendications 1-4, dans lequel l'aiguille d'infusion (51) et le raccord de tubulure d'insertion (52) sont formés en une seule pièce en matière plastique ;
le bord d'ouverture (56) de l'extrémité distale de l'aiguille d'infusion (51) étant configuré de sorte à être orthogonal à un centre axial (57) de l'aiguille d'infusion (51), et le côté périphérique intérieur et le côté périphérique extérieur du bord d'ouverture (56) étant formés au moment du moulage en surfaces arrondies (59, 60) s'étendant vers l'extérieur.

6. Connecteur à usage médical selon l'une quelconque des revendications 1-5, dans lequel l'aiguille d'infusion (51) est enduite d'un lubrifiant appliqué sur cette dernière.

7. Cathéter à demeure comprenant :
le connecteur selon l'une quelconque des revendications 1-6,
dans lequel :
un passage traversant (14) est ménagé dans le raccord de cathéter (6) du corps de canule (1) sensiblement dans la direction axiale ;
une partie proximale du passage traversant (14) est formée en l'orifice de connexion;
un cathéter (5) est inséré dans une partie distale du passage traversant (14) ;
le cathéter (5) est disposé sensiblement dans la direction axiale, et est formé en un corps creux allongé ouvert à ses extrémités distale et proximale ;
le cathéter (5) est mis en communication avec l'aiguille d'infusion (51) lorsque l'aguille d'infusion (51) est insérée dans le bouchon (8).
